# FASCICULE DE BREVET EUROPEEN

(11) **EP 1 800 618 B1**
(45) Date de publication et mention de la délivrance du brevet: **24.06.2009**
(21) Numéro de dépôt: 06126702.7
(22) Date de dépôt: 20.12.2006
(51) Int. Cl.: A61F 2/00

(54) **Dispositif chirurgical formant prothèse chirurgicale destiné au soutènement d'un organe biologique d'un mammifère**
Chirurgische Prothese zum Stützen eines Organs
Surgical prosthesis forming device used to implant an organ support in a mammal

(30) Priorité: 26.12.2005 FR 0554088; 16.06.2006 US 804972 P
(43) Date de publication de la demande: 27.06.2007
(73) Titulaire: Bouffier, Bernard, 25480 Ecole (FR)
(72) Inventeur: Bouffier, Bernard, 25480 Ecole (FR)
(74) Mandataire: Portal, Gérard

(56) Documents cités:
- WO-A-20/04004600
- US-A- 6 110 101
- US-A1- 2003 065 402
- US-A1- 2004 015 048

## Description

La présente invention concerne essentiellement un dispositif chirurgical formant prothèse chirurgicale, pour réaliser l'implantation d'un soutènement d'un organe d'un mammifère.

Dans le cadre de l'invention, tout organe d'un mammifère, pour lequel le soutènement peut être réalisé, peut être traité par le dispositif chirurgical selon l'invention.

L'invention est particulièrement adaptée au soutènement des organes pelviens, de l'urètre, de la vessie, du vagin, du col de l'utérus, de l'utérus et du rectum.

De préférence, l'invention concerne un dispositif chirurgical formant prothèse chirurgicale pour réaliser un soutènement urétral pour le traitement de l'incontinence urinaire, en particulier d'un mammifère femelle, de préférence une femme, ou d'un mammifère mâle, de préférence un homme . de préférence, ce dispositif doit être suffisamment versatile pour être utilisable sans modification significative à la fois dans l'incontinence urinaire femelle et mâle.

Ainsi, le dispositif formant prothèse chirurgicale selon la présente invention est prévu pour fixer ou maintenir des tissus ou des organes différents, en apportant éventuellement des modifications plus significatives, imposées par les conditions anatomiques ou pathologiques.

### ETAT DE LA TECHNIQUE

On sait que l'incontinence urinaire d'effort est la conséquence d'une mobilité excessive de l'urètre lors d'une pression abdominale importante, provoquée par la toux, le rire, la course, plus généralement une poussée abdominale.

Les fuites d'urine surviennent d'autant plus facilement et abondamment que la mobilité urétrale est importante, voire que le canal est ptosé en permanence.

L'incontinence peut être associée à un prolapsus des organes pelviens (vagin, utérus, vessie, rectum), dans des proportions variables. Elle peut être isolée. Elle est presque toujours la conséquence plus ou moins tardive des accouchements par voie basse. Elle réalise une redoutable infirmité, atteignant douloureusement à la liberté et à la dignité des femmes et des hommes. Elle est volontiers tenue secrète, ce qui rend aléatoire les études de morbidité.

Il a déjà été proposé par une invention précédente du présent inventeur, publiée sous le numéro FR-A-2 843 876, un dispositif chirurgical comprenant un dispositif de fixation formant cage, coopérant avec un fil de traction lui-même solidarisé à un élément de soutènement en forme de bandelette, clairement visible aux figures 1 et 3 de ce document. Le dispositif de fixation formant cage est introduit à l'aide d'un trocart à l'état replié au-dessus de la paroi pelvienne transversale, et après retrait du trocart, et son déploiement, une traction sur le fil de traction aboutit à un écrasement de la cage qui prend ainsi une forme aplatie procurant une grande surface d'appui sur la paroi pelvienne, en assurant ainsi une fixation sûre et fiable contre la paroi pelvienne sans risque de déchirement(s) au cours du temps.

Les résultats fonctionnels de ce dispositif constituent une amélioration significative par rapport aux solutions techniques antérieures en limitant ou quasi limitant les risques de complications graves.

Cependant, il est apparu que le système de cage est relativement coûteux à fabriquer, et nécessite un apprentissage des praticiens pour une mise en place correcte, notamment dans la phase délicate de déploiement de la cage et de son écrasement par action de traction sur le fil de traction.

Un dispositif chirurgical selon le préambule de la revendication 1, est connu du document US-A-6 110 101.

### BUTS DE L'INVENTION

La présente invention a pour but principal de résoudre le nouveau problème technique consistant en la fourniture d'un dispositif chirurgical formant prothèse chirurgicale de fixation et de soutien d'un organe biologique, notamment pour le soutènement urétral pour le traitement de l'incontinence urinaire, en particulier d'un mammifère femelle, de préférence une femme, qui soit moins agressif que les dispositifs antérieurs, qui soit d'une dimension réduite et utilise moins de pièces en étant ainsi d'une conception simplifiée.

La présente invention a encore pour but principal de résoudre le nouveau problème technique précité avec un dispositif chirurgical particulièrement adapté au soutènement des organes pelviens, de l'urètre, de la vessie, du vagin, du col de l'utérus, de l'utérus et du rectum.

La présente invention a encore pour but principal de résoudre ces nouveaux problèmes techniques selon un dispositif qui permet la mise en oeuvre d'un procédé chirurgical de fixation et de soutien selon une procédure d'intervention simple, nécessitant un nombre minimum d'étapes, rapide, sous anesthésie locale, sans hospitalisation, et sans immobilisation post-opératoire, donc réalisable en «ambulatoire».

L'invention permet pour la première fois de résoudre ces problèmes techniques d'une manière simple, fiable, peu coûteuse, reproductible à l'échelle industrielle et médicale, en facilitant la procédure chirurgicale en permettant une intervention simplifiée, rapide, sous anesthésie locale, sans hospitalisation, et sans immobilisation post-opératoire.

### DESCRIPTION DE L'INVENTION

Ainsi, selon premier aspect, la présente invention fournit un dispositif chirurgical formant prothèse chirurgicale, destiné au soutènement d'un organe biologique à soutenir d'un mammifère, notamment à traiter l'incontinence urinaire, caractérisé en ce qu'il comprend :
a) au moins un premier élément de soutènement comprenant un élément de forme allongée définissant une première et une deuxième extrémité pour exercer une action de soutènement dudit organe biologique ; ledit premier élément de forme allongée étant réalisé au moins en partie en un matériau sensiblement non extensible, souple, déformable ;
b) au moins un premier élément de traction, avantageusement filiforme, définissant une première extrémité, dite distale, et une deuxième extrémité, dite proximale, pouvant être rendue solidaire par ladite extrémité proximale au moins provisoirement avec au moins une extrémité dudit premier élément de soutènement allongé, ladite extrémité distale étant prévue pour constituer au moins un premier système de fixation ou d'ancrage activé par une traction exercée sur le premier élément de traction notamment au niveau de son extrémité proximale ;
ledit système de fixation prévu à l'extrémité distale de l'élément de traction comprend au moins un repli, de préférence plusieurs replis, de l'élément de traction qui, lors d'une action de traction exercée sur l'élément de traction, est déformé ou écrasé pour se déployer en imitant un effet parapluie définissant une surface suffisante de fixation ou d'ancrage, On entend par «surface suffisante de fixation ou d'ancrage», une surface qui assure une fixation ou un ancrage sûr et fiable de l'extrémité distale du premier élément de traction contre la paroi ou les tissus résistants du corps du mammifère, sans risque de déchirement de ladite paroi ou desdits tissus lors de ladite traction, ou au cours du temps alors que le mammifère mène une vie sans précaution particulière.

Selon un autre mode de réalisation avantageux de l'invention, le premier élément de traction est aussi réalisé au moins en partie en un matériau sensiblement non extensible, souple, déformable. Selon une variable de réalisation particulière, le premier élément de traction peut être réalisé en le même matériau que celui utilisé pour réaliser le premier élément de soutènement.

Selon encore un autre mode de réalisation avantageux de l'invention, on peut prévoir un deuxième élément de traction, de préférence de même conception que le premier élément de traction, ainsi également filiforme, définissant aussi une première extrémité, dite distale, et une deuxième extrémité, dite proximale, pouvant être rendu solidaire par ladite extrémité proximale au moins provisoirement avec au moins une autre extrémité dudit premier élément de soutènement allongé, ladite extrémité distale du deuxième élément de traction étant aussi prévue pour constituer au moins un deuxième système de fixation ou d'ancrage sur une autre paroi ou une autre partie de paroi ou des tissus résistants du corps du mammifère, également activé par une traction exercée sur le deuxième élément de traction, notamment au niveau de son extrémité proximale.

Selon un mode de réalisation particulièrement avantageux de l'invention, le matériau sensiblement non extensible, souple, déformable, utilisé pour réaliser soit le premier élément de traction, soit le deuxième élément de traction, soit chaque élément de traction, soit le ou les élément(s) de soutènement, soit l'ensemble, est un polymère organique compatible avec une implantation à l'intérieur des tissus d'un mammifère, ce matériau organique étant avantageusement choisi parmi le polyéthylène, le polypropylène, ou le nylon. Le matériau actuellement préféré est le polypropylène.

Le choix de ce matériau est particulièrement avantageux car il est disponible commercialement à un prix raisonnable sous la forme de fils qui peuvent être tissés ou tricotés pour se présenter sous la forme d'une bandelette dont la longueur et la largeur peuvent être définie(s) de manière indépendante, à volonté, et ainsi être adaptée(s) pratiquement à chaque patient. De telles bandelettes sont généralement disponibles dans le commerce et peuvent être découpées à volonté directement par le chirurgien.

Ainsi, selon une variante de réalisation particulière, on peut prévoir que le premier élément de soutènement, ou chaque élément de soutènement, présente la forme d'une bandelette ayant une largeur suffisante pour réaliser un soutènement sûr et fiable de l'organe à soutenir. On entend par largeur suffisante de l'élément de soutènement, une largeur qui assure le soutènement de l'organe à soutenir sans risque de rupture ou de blessure de cet organe comme cela pourrait être le cas si la largeur de l'élément de soutènement est trop faible.

Selon une autre variante de réalisation particulière de l'invention, on peut prévoir que le premier élément de traction ou le deuxième élément traction, ou les deux, comprenne, ou soit constitué d'une bandelette de largeur plus faible que la largeur de la bandelette de l'élément de soutènement. En particulier, cette largeur de la bandelette de l'élément de traction peut être inférieure ou égale à environ la moitié de la largeur de la bandelette de l'élément de soutènement.

On comprend que, selon une autre variante de réalisation de l'invention, l'élément de soutènement peut être réalisé monobloc avec l'un des deux éléments de traction précité. Ceci est particulièrement aisé puisque dans le mode de réalisation sous forme de bandelettes, il suffira de tricoter en une seule étape l'élément de soutènement à la suite de l'élément de traction à une extrémité de ce dernier destinée à être l'extrémité proximale, une partie plus large destinée à constituer la partie élément de soutènement.

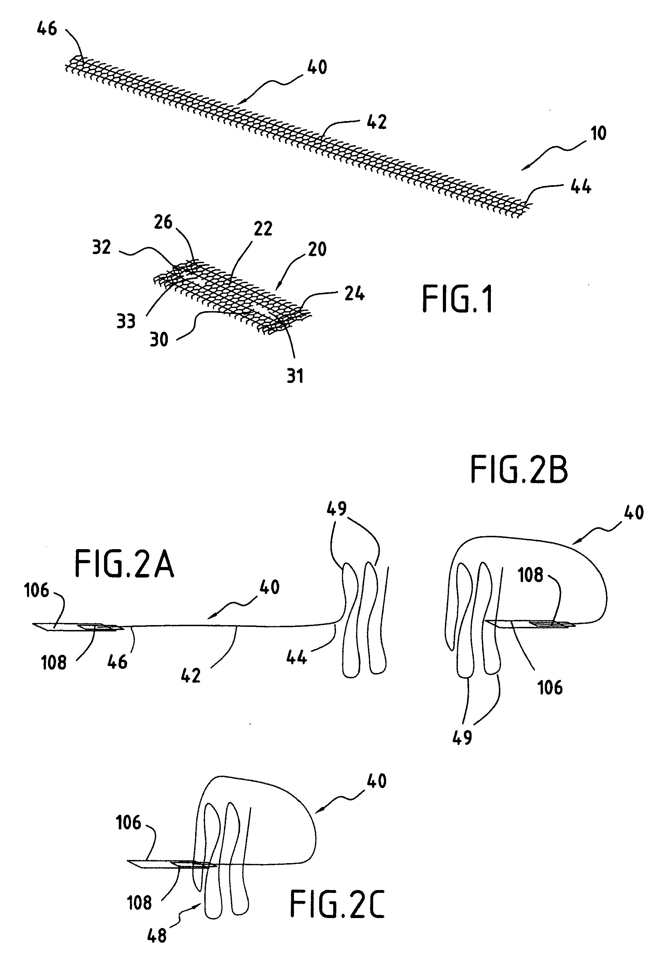

Avec ce mode de réalisation de chaque élément de traction sous forme de bandelette qui peut être réalisée sous forme tissée ou non tissée par les techniques textiles habituelles à partir de fils, il est particulièrement aisé de réaliser le système de fixation précité en formant plusieurs replis transversaux d'une extrémité de la bandelette, comme pour le repliage d'une serviette, et en les solidarisant ensemble par le passage d'un élément formant aiguille qui peut être solidarisé momentanément ou en permanence à l'autre extrémité de la bandelette, comme cela sera décrit plus loin en référence aux figures 2A, 2B, et 2C.

Selon encore une autre variante de réalisation particulière de l'invention, il est prévu au niveau de chaque extrémité de chaque élément de soutènement au moins un élément de solidarisation d'au moins une extrémité proximale d'au moins un élément de traction. Selon une réalisation particulière avantageuse de l'invention, cet élément de solidarisation comprend, ou est constitué de, une fente réalisée dans le matériau de l'élément de soutènement, cette fente étant selon une réalisation particulière disposée sensiblement parallèlement aux bords longitudinaux de ladite bandelette. La dimension de cette fente peut varier dans de larges limites. Cependant, la dimension de cette fente sera limitée au minimum pour permettre le passage de l'extrémité proximale de l'élément de traction en vue de sa solidarisation avec l'élément de soutènement. D'autre part, pour ne pas fragiliser les extrémités de la bandelette, on peut réaliser un repli des extrémités de la bandelette pour former par exemple une double épaisseur, comme visible à la figure 1, en vue de les renforcer mécaniquement.

La solidarisation de l'élément de traction avec l'élément de soutènement peut être réalisée très simplement, lorsque l'élément de traction et l'élément de soutènement sont formés chacun d'une bandelette non tissée ou tissée réalisée à partir de fils de matériau organique polymère précité, après passage de ladite fente, par repli et application de l'élément de traction contre la surface en regard de l'élément de soutènement, en réalisant une solidarisation de leurs surfaces respectives par imbrication naturelle en obtenant un effet de fixation de type Velcro, bien connu de l'homme de l'art.

Selon une autre variante de réalisation de l'invention, l'élément de soutènement peut comprendre un élément de solidarisation, qui n'est pas réalisé sous forme d'une fente, mais sous forme d'un orifice de forme quelconque, et en particulier le plus simplement de forme circulaire.

Par ailleurs, selon un autre mode de réalisation avantageux du dispositif selon l'invention, celui-ci est prévu pour un traitement de l'incontinence urinaire, en particulier d'un mammifère femelle, en particulier une femme, ou d'un mammifère mâle, en particulier un homme. La procédure de fixation des dispositifs selon l'invention dans le cas d'un mammifère femelle ou d'un mammifère mâle sera décrite plus loin dans le cadre de la procédure chirurgicale. Il est à noter que lorsque le dispositif de selon l'invention est prévu pour le soutènement du bulbe urétral d'un mammifère mâle, il est préféré que l'élément de soutènement soit d'une largeur plus grande que dans le cadre du soutènement de l'urètre d'un mammifère femelle.

Selon un deuxième aspect, la présente invention concerne aussi en tant que produit nouveau indépendamment brevetable chaque élément de traction comprenant une extrémité distale comprenant ou constituant un système de fixation ou d'ancrage, tel que précédemment défini ou tel que résultant de la description suivante faite en référence aux dessins qui font partie intégrante de la présente invention et qui complètent donc la présente description.

Selon un troisième aspect, la présente invention couvre encore un kit de soutènement et de fixation ou d'ancrage, caractérisé en ce qu'il comprend au moins un dispositif chirurgical formant prothèse chirurgicale selon l'un quelconque des aspects précédents, ainsi qu'un dispositif introducteur avantageusement sous forme d'un trocart de pénétration, avec présence d'un élément poussoir de l'élément de traction avec son extrémité distale comprenant ou constituant le système de fixation ou d'ancrage, monté de manière compacte ou repliée à l'intérieur du trocart de pénétration. Selon une variante de réalisation, le trocart ou l'élément poussoir comporte au moins un élément de blocage anti-retrait de l'élément de traction en place à l'intérieur du trocart. Le trocart a par exemple un diamètre de 4 mm.

Selon un quatrième aspect, la présente invention couvre encore une méthode chirurgicale pour réaliser le soutènement d'un organe à soutenir d'un mammifère en ayant besoin, caractérisée en ce qu'il comprend :
a) une anesthésie locale au voisinage de l'organe à soutenir sur le futur trajet de la prothèse ;
b) une incision et une dissection des tissus en regard et de part et d'autre de l'organe à soutenir dudit mammifère ;
c) la mise en place d'un premier dispositif introducteur formant trocart dans le passage créé par ladite incision et dissection des tissus, sur un côté et au-delà de l'organe à soutenir, et après franchissement par perforation à l'aide du dispositif introducteur du support relativement résistant au déchirement dudit mammifère, tel que os ou tissus appropriés comme par exemple la paroi abdominale ou pelvienne transversale, ou encore la membrane musculaire du trou obturé du bassin, ledit dispositif introducteur formant trocart ayant une forme tubulaire creuse comprenant en son intérieur un premier élément de traction précité, ainsi qu'un élément poussoir;
d) une action de poussée sur l'élément poussoir de manière à faire sortir du dispositif introducteur l'extrémité du premier élément de traction jusqu'à ce que au moins le système de fixation de l'élément de traction soit libéré au-delà dudit support relativement résistant au déchirement ;
e) après la libération ou largage du système de fixation, une traction au voisinage de la partie proximale de l'élément de traction, permettant le positionnement et l'ouverture ou déploiement par effet d'écrasement de l'extrémité distale de l'élément de traction formant système de fixation ou d'ancrage, avec obtention d'un positionnement dit en parapluie présentant une grande surface de fixation ou d'ancrage contre ledit support relativement résistant au déchirement dudit mammifère ;
f) le retrait du trocart de pénétration laissant en place le système d'ancrage s'appuyant sur le support choisi, pouvant ainsi jouer son rôle de point de fixation car la taille du système de fixation ou d'ancrage excède largement celle de l'orifice provoqué par la pénétration du trocart ;
g) la répétition des étapes précédentes c), d), e), f) de l'autre côté de l'organe pour introduire un deuxième élément de traction, de préférence identique au premier élément de traction ;
h) lorsque aucun élément de traction n'est initialement prévu solidaire de l'élément de soutènement, la solidarisation de l'extrémité proximale du premier ou deuxième élément de traction avec un élément de solidarisation prévu au voisinage d'une première extrémité d'un élément de soutènement allongé, de manière à réaliser un premier mouvement de traction sur l'extrémité proximale de l'élément de traction pour un pré-positionnement ;
i) la réalisation de la même procédure que les étapes précédentes concernant l'autre deuxième ou premier élément de traction avec un autre élément de solidarisation prévu au voisinage d'une deuxième extrémité du même élément de soutènement allongé, jusqu'à mise en tension dudit élément de soutènement allongé en position appropriée avec ledit organe pour réaliser le soutien recherché dudit organe ;
j) la fermeture ou suture de l'incision avec un moyen de fermeture ou de suture chirurgicale appropriée, tel que fil ou agrafe résorbable.

Selon un premier mode de réalisation préféré de ce procédé chirurgical, celui-ci est utilisé pour réaliser le traitement de l'incontinence d'un mammifère femelle, en particulier d'une femme. Dans ce cadre, l'organe à soutenir est l'urètre, de préférence au voisinage de la partie urétrale proche de la vessie, Dans ce cadre, il est préféré selon la présente invention que l'extrémité distale de l'élément de traction prenne appui sur les tissus de la paroi pelvienne, de préférence des tissus sensiblement transversaux de la paroi pelvienne dans la partie située entre l'urètre et les côtés du mammifère femelle, en particulier une femme, au-dessus de la paroi vaginale.

Selon un second mode de réalisation préféré de ce procédé chirurgical, celui-ci est utilisé pour réaliser le traitement de l'incontinence d'un mammifère mâle, en particulier d'un homme. Dans ce cadre, l'organe à soutenir est encore l'urètre, de préférence au voisinage du bulbe urétral. Dans ce cadre, il est préféré, selon la présente invention, que l'extrémité distale de l'élément de traction prenne appui sur les tissus de la membrane musculaire obturant l'orifice obturé du bassin, avantageusement de façon bilatérale.

On comprend que dans le cadre de la présente invention, on évite le système d'ancrage en forme de cage qui était le constituant essentiel du dispositif chirurgical prévu dans la solution antérieure de l'inventeur décrite dans le document FR-A-2 843 876, auquel l'homme de l'art pourra utilement se reporter.

Le dispositif chirurgical selon la présente invention est particulièrement simple puisqu'il utilise en pratique et selon un mode de réalisation considéré actuellement comme particulièrement avantageux, un concept unique de bandelette(s) largement disponible(s) dans le commerce à des différentes dimensions et caractéristiques de produits à maillage variable, tissés ou non tissés, et à un prix raisonnable. D'autre part, il peut être utilisé indifféremment pour traiter l'incontinence femelle ou féminine ; ou mâle où masculine.

D'autres buts, caractéristiques et avantages de l'invention apparaîtront clairement à partir de la description explicative et qui va suivre faite en référence aux dessins annexés représentant un mode de réalisation actuellement préféré de l'invention donné simplement à titre d'illustration et qui ne saurait donc en aucune façon limiter la portée de l'invention.

### DESCRITION DES FIGURES

Dans les dessins :
- la figure 1 représente de manière éclatée, les éléments principaux du dispositif chirurgical formant prothèse chirurgicale selon un mode de réalisation actuellement préféré de l'invention comprenant au moins un premier élément de soutènement 20 de forme allongée, par exemple ici sous forme d'une bandelette ; au moins un premier élément de traction 40 avantageusement filiforme, dont l'extrémité distale sera modifiée pour montré aux figures 2A, 2B, 2C, pour comprendre ou constituer un premier système de fixation ou d'ancrage 48 ;
- les figures 2A, 2B, 2C représentent les étapes successives de fabrication d'un système de fixation ou d'ancrage 48 à l'extrémité 44 de chaque élément de traction, ici portant le numéro de référence générale 40 ;
- la figure 2A montre la première étape selon laquelle l'extrémité 46 de l'élément de traction 40 est pourvue d'un outil de solidarisation 106, par exemple sous forme d'un élément formant aiguille, tandis que plusieurs plis 49 sont formés sur l'autre extrémité 44, comment représenté, chaque pli ayant une longueur suffisante pour pouvoir constituer un système de fixation d'ancrage 48 sûr et fiable ;
- la figure 2B présente la deuxième étape montre le début de la procédure de perforation des plis 49 sensiblement en leur milieu par l'outil de solidarisation 106 ;
- la figure 2C montre la troisième et dernière étape de la procédure de perforation des plis constituant alors le système de fixation ou d'ancrage 48, par l'outil 106 de sorte que l'an constate que ce système 48 peut être simplement réalisé selon un principe de couture à l'aide d'un élément formant aiguille ;

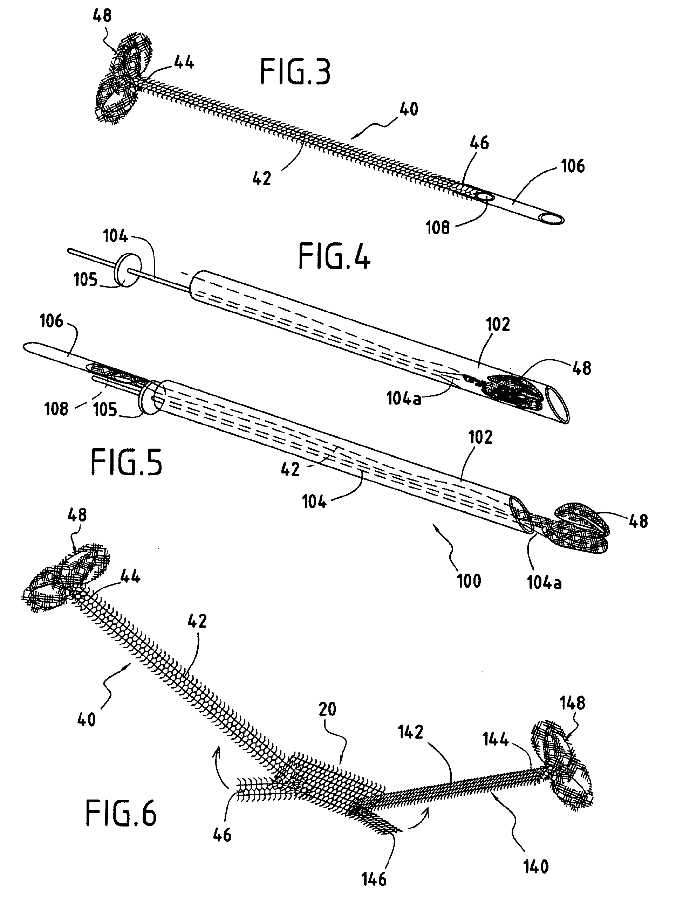

- la figure 3 représente de manière agrandie l'élément de traction montré à la figure 2C, servant ici à la fois pour constituer le premier élément de traction et le deuxième élément de traction, avec son extrémité proximale ou libre 46 de l'élément de traction 40 montée à l'intérieur d'un outil de solidarisation 106 en forme d'aiguille tubulaire, pourvu d'un élément de blocage en position 108 , et avec son extrémité distale 44 présentant les replis constituant alors le système de fixation ou d'ancrage 48
- la figure 4 représente schématiquement l'ensemble du dispositif introducteur 100, comprenant d'une part un trocart 102, dans lequel est inséré de l'élément traction 40 ou 140 avec son outil de solidarisation 106 ; et un élément poussoir 104 évitant un retrait de la bandelette ;
- la figure 5 représente de manière schématique, l'étape de poussée de l'extrémité 44 de l'élément de traction 40 de manière à expulser le dispositif de fixation d'ancrage 48, l'élément poussoir pouvant présenter à cet effet une extrémité 104a par exemple sous forme de fourches permettant de pousser de manière sûre et fiable le système de fixation 48 à l'extérieur du trocart 102 ;
- la figure 6 représente schématiquement la solidarisation d'un premier élément de traction 40 et d'un deuxième élément de traction 140 avec l'élément de soutènement 20, ici par insertion dans chaque fente respective 31,32, solidarisation par repliage et application de l'extrémité proximale libre de chaque élément de traction contre la surface soit de l'élément de soutènement, soit comme représenté ici de l'élément de traction ;

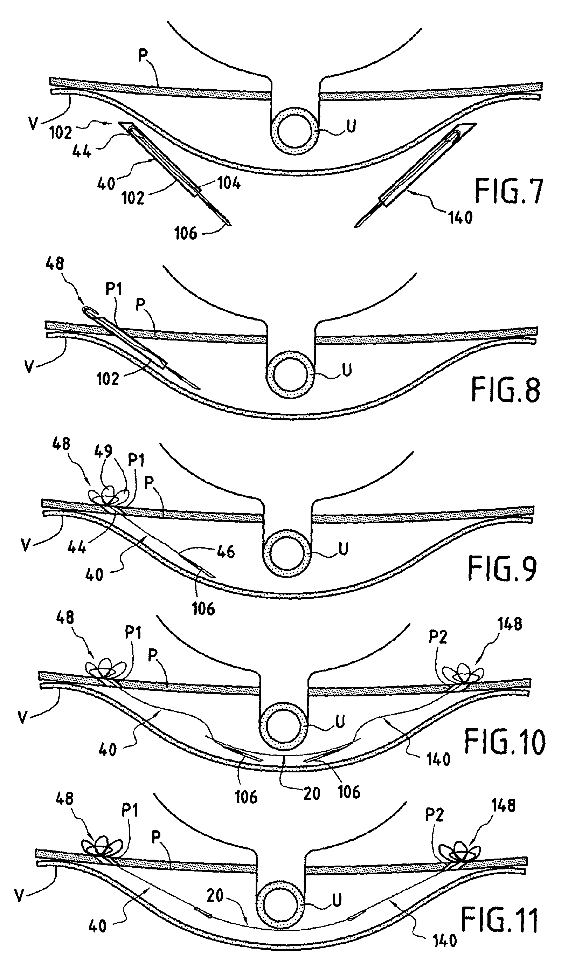

- la figure 7 représente symboliquement les deux éléments de traction 40, 140 à mettre en place de part et d'autre de l'organe à soutenir, ici pour le traitement de l'incontinence féminine avec de ce fait soutien de l'urètre U ;
- la figure 8 représente symboliquement l'insertion du premier élément de traction avec passage au travers d'une incision et dissection réalisée au travers d'un support relativement résistant au déchirement d'un mammifère, tel que os ou ici tissus de la paroi pelvienne P ;
- la figure 9 représente symboliquement l'étape de traction permettant de solidariser de manière sûre et fiable l'extrémité distale de l'élément de traction contre le support relativement résistant au déchirement du mammifère ici la paroi pelvienne ;
- la figure 10 représente symboliquement l'instant où le chirurgien a réalisé le placement du deuxième dispositif de traction 140, et la solidarisation du premier dispositif de traction et du deuxième dispositif de traction au premier dispositif de soutènement 20, avant le réglage de la traction pour réaliser le soutien approprié de l'organe à soutenir, ici l'urètre U;
- la figure 11 représente symboliquement la fin de la procédure chirurgicale au moment où le chirurgien a terminé de solidariser les éléments de traction 40, 140 avec l'élément de soutènement 20 après réglage de la traction permettant de réaliser le soutien approprié de l'organe à soutenir, ici l'urètre ;

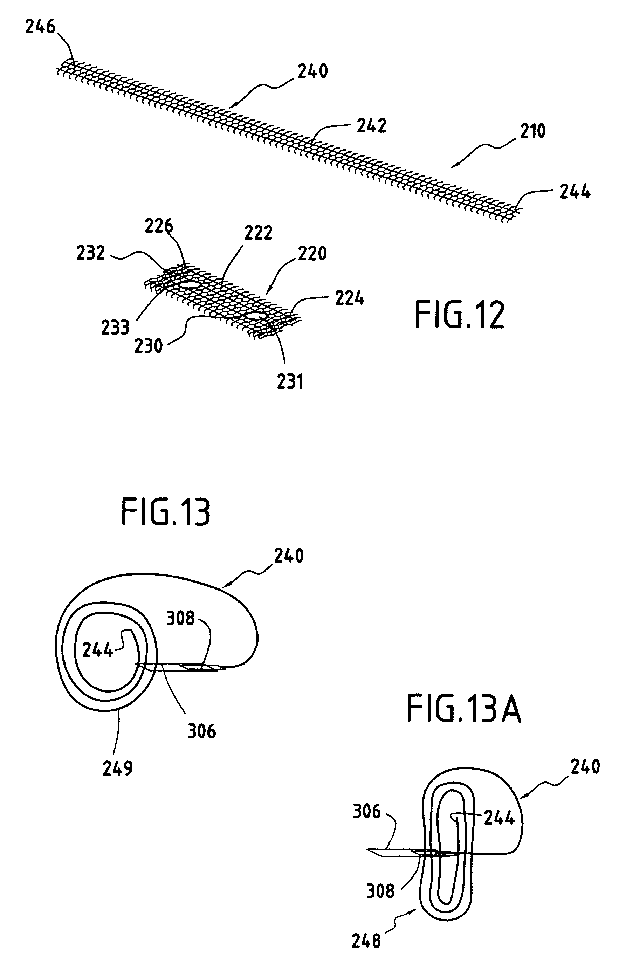

- la figure 12 représente de manière éclatée un deuxième mode de réalisation des éléments principaux du dispositif chirurgical selon la présente invention comprenant au moins un premier élément de soutènement 220, de forme allongée, par exemple ici sous forme d'une bandelette, et au moins un premier élément de traction 240 avantageusement filiforme, dont l'extrémité distale sera modifiée comme montré aux figures 13, 13A, pour comprendre ou constituer un premier système de fixation ou d'ancrage 248; de manière similaire au mode de réalisation des figures 1 à 6;
- les figures 13, et 13A, représentent les étapes successives de fabrication d'un système de fixation ou d'ancrage 248 à l'extrémité 244 de chaque élément de traction , ici portant le numéro de référence générale 240 de manière similaire aux Figures 2A à 2C;

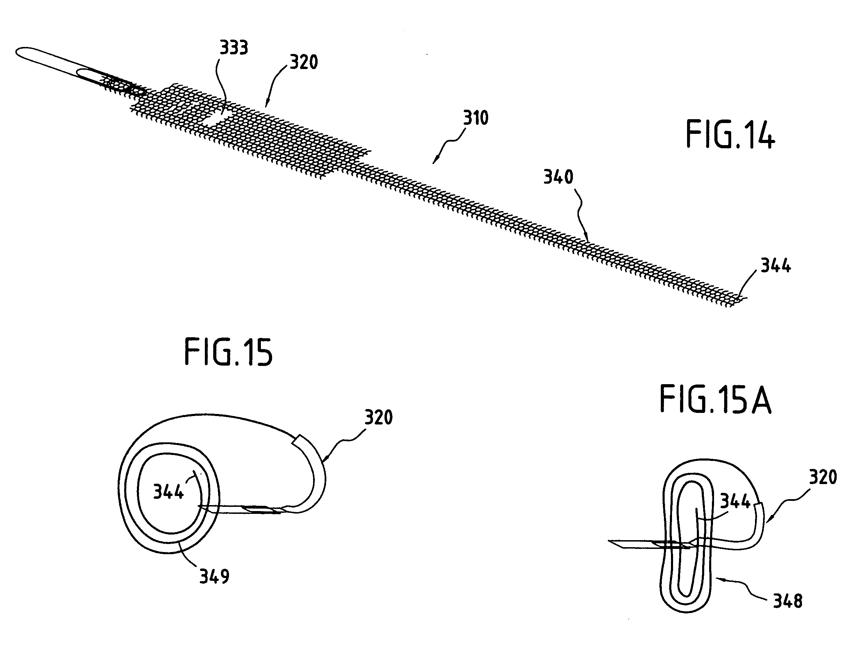

- la figure 14 présente un troisième mode de réalisation d'un dispositif chirurgical selon la présente invention, dans lequel l'élément de soutènement 320 de forme allongée, par exemple ici sous la forme d'une bandelette, est monobloc avec un premier élément de traction 340 de manière similaire à celui des deux premiers modes de réalisation représentés aux figures 1 et 12. Dans ce cadre, on comprend que l'élément de soutènement est réalisé en une seule étape lors de la fabrication de l'élément de traction en continuité avec celui-ci ;
- les figures 15, et 15A, représentent les étapes successives de fabrication d'un système de fixation ou d'ancrage 348 à l'extrémité 344 de chaque élément de traction 340 de manière similaire aux Figures 2A à 2C;

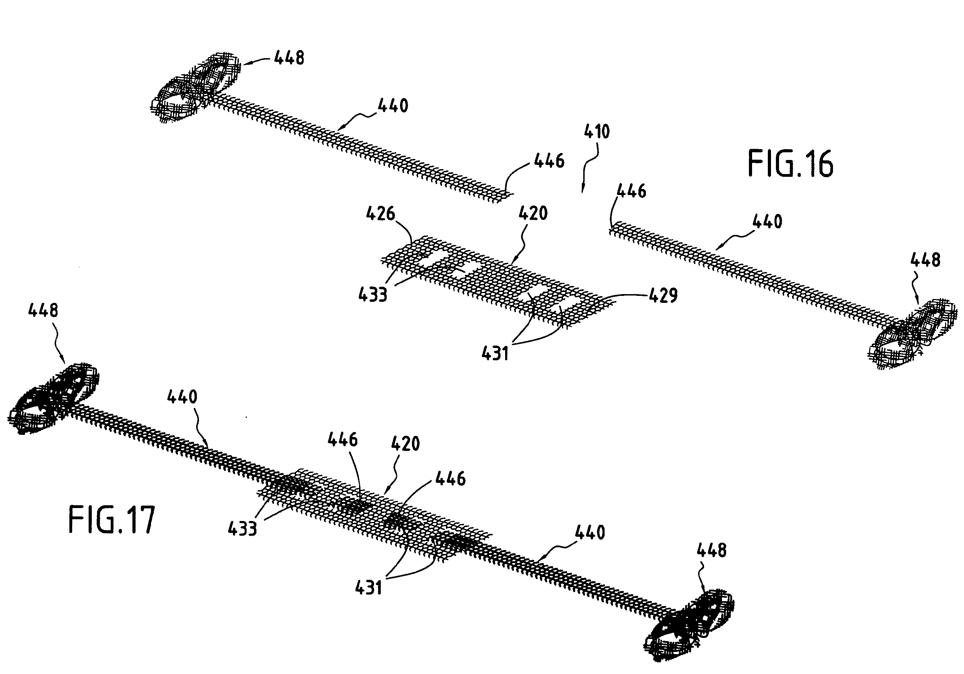

- la figure 16 représente, de manière éclatée, un quatrième mode de réalisation d'un dispositif chirurgical selon la présente invention, dans lequel l'élément de soutènement 420 est particulièrement adapté pour réaliser le soutènement du bulbe urétral d'un mammifère mâle, de préférence un homme, dans lequel l'élément de soutènement est de plus grande largeur, et comprend ici de préférence de chaque côté au moins un et encore de préférence au moins deux organes de solidarisation, tels que orifices ou fentes, de manière à simplifier la procédure de solidarisation de l'élément de soutènement avec les éléments de traction 440, identiques ou similaires à ceux objets des modes de réalisation précédents représentés aux figures 1 à 15 ;
- la figure 17 représente le dispositif chirurgical de la figure 16 sous forme assemblée, permettant de voir la facilité d'assemblage ;

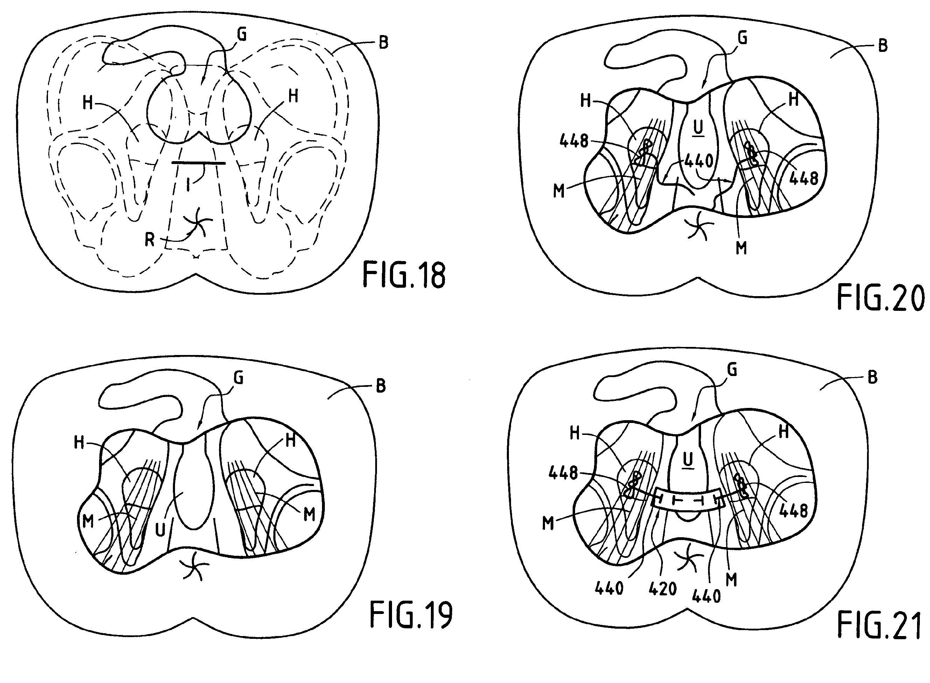

- la figure 18 représente la première étape de la procédure chirurgicale d'implantation chez un mammifère mâle, de préférence un homme, du dispositif chirurgical représenté à la figure 16, montrant la ligne d'incision transversale I entre le rectum et les parties génitales, le plus loin possible du rectum ;
- la figure 19 représente, après dégagement des chairs , les trous obturateurs du bassin, avec de manière schématique chaque membrane musculaire obturant chaque trou obturateur du bassin ;
- la figure 20 représente chaque système de fixation 440 de la figure 16 en place après perforation de la membrane musculaire au niveau du trou obturateur, à l'aide d'un trocart perforateur, de manière similaire à la mise en place chez un mammifère femelle comme représenté aux figures 7 à 9 ;
- la figure 21 représente la fin de la mise en place ce dispositif chirurgical selon l'invention, après solidarisation de l'élément de soutènement 420 avec les éléments de traction 440 de manière similaire à la position de solidarisation représentée à la figure 17, et traction selon le niveau de traction souhaité par le chirurgien ;

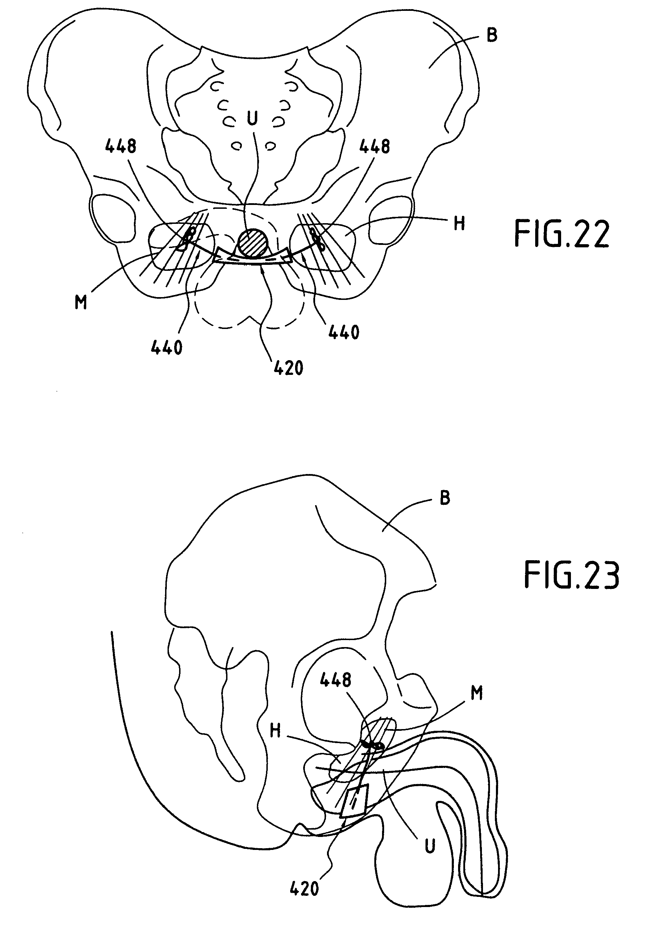

- la figure 22 représente une vue de face du bassin, représentant en coupe le bulbe urétral au niveau du soutien avec un dispositif chirurgical selon l'invention des figures 16 à 21 ; et
- la figure 23 représente une vue de côté du bassin, représentant le dispositif chirurgical selon l'invention des figures 16 à 21 en position de soutien, permettant de bien voir le système de fixation et d'ancrage 448 prenant appui, ici en forme de parapluie, sur la membrane musculaire M obturant l'orifice obturé H du bassin, à l'intérieur de l'orifice, donc à l'opposé de l'élément de soutènement 440.

En référence aux figures 1 à 11, et en particulier aux figures 1 à 6, un dispositif chirurgical formant prothèse chirurgicale, selon la présente invention est représenté par le numéro de référence générale 10.

Ce dispositif 10 est destiné au soutènement d'un organe biologique U à soutenir d'un mammifère, notamment ici à traiter l'incontinence urinaire par le soutien de l'urètre référencée U, et est caractérisé en ce qu'il comprend :
a) au moins un premier élément de soutènement 20 comprenant un élément de forme allongée 22 définissant une première extrémité 24 et une deuxième extrémité 26 pour exercer une action de soutènement dudit organe biologique ; ledit premier élément de forme allongée étant réalisé au moins en partie en un matériau sensiblement non extensible, souple, déformable ;
b) au moins un premier élément de traction 40 , avantageusement filiforme 42, définissant une première extrémité 44 dite distale et une deuxième extrémité 46 , dite proximale, pouvant être rendu solidaire par ladite extrémité proximale 46 au moins provisoirement avec au moins une extrémité 26 dudit premier élément de soutènement 20 allongé, ladite extrémité distale 44 étant prévue pour constituer au moins un premier système de fixation ou d'ancrage 48, voir figures 2C et 3, activé par une traction exercée sur le premier élément de traction 40 notamment au niveau de son extrémité proximale 46 après son insertion dans une première zone telle que P1.

Selon un mode de réalisation avantageux de l'invention, représenté aux figures 2A, 2B, 2C et 3 ledit système de fixation 48 prévu à l'extrémité distale 44 de l'élément de traction 40 comprend au moins un repli, en pratique plusieurs replis, 48 de l'élément de traction 40 qui, lors d'une action de traction exercée sur élément de traction, est déformé ou écrasé pour se déployer en imitant un effet parapluie définissant une surface suffisante de fixation ou d'ancrage. On entend par « surface suffisante de fixation ou d'ancrage », une surface qui assure une fixation ou un ancrage sûr et fiable de l'extrémité distale du premier élément traction contre la paroi ou les tissus résistants P du corps du mammifère, sans risque de déchirement de ladite paroi ou desdits tissus lors de ladite traction, ou au cours du temps alors que le mammifère mène une vie sans précaution particulière.

Comme indiqué précédemment, la figure 2A montre la première étape de fabrication du système de fixation d'ancrage 48, selon laquelle l'extrémité 46 de l'élément de traction 40 est tout d'abord pourvue d'un outil de solidarisation 106, par exemple sous forme d'un élément formant aiguille sensiblement tubulaire, pourvu d'un élément de blocage en position 108 de l'extrémité 46 de l'élément de traction. On réalise à l'autre extrémité 44 la formation de plusieurs plis 49, comme représenté, chaque pli 49 ayant une longueur suffisante pour pouvoir constituer un système de fixation ou d'ancrage 48 sûr et fiable. En général, chaque pli pourra avoir par exemple une longueur d'environ 3 cm, à comparer avec la largeur de l'élément de traction qui ne dépassera généralement pas 0,5 cm.

La figure 2B présente la deuxième étape montrant le début de la procédure de perforation des plis 49 sensiblement en leur milieu par l'outil de solidarisation 106.

La figure 2C montre la troisième et dernière étape de la procédure de perforation des plis constituant alors le système de fixation ou d'ancrage 48, par l'outil 106 de sorte que l'on constate que ce système de fixation ou d'ancrage 48 peut être simplement réalisé selon un principe de couture à l'aide d'un élément formant aiguille.

Pour faciliter le passage de l'aiguille, on peut prévoir une fente ou un orifice dans les plis que l'on réalise par tous moyens, comme par exemple un poinçon.

Selon un autre mode de réalisation avantageux de l'invention, le premier élément de traction 40, est réalisé au moins en partie en un matériau sensiblement non extensible, souple, déformable. Selon une variable de réalisation particulière, le premier élément de traction 40 peut être réalisé en le même matériau que celui utilisé pour réaliser le premier élément de soutènement 20.

Selon encore un autre mode de réalisation avantageux de l'invention, on peut prévoir un deuxième élément de traction, de préférence de même conception que le premier élément de traction, qui porte ainsi les mêmes chiffres de référence que le premier élément de traction, augmentés de 100, donc ici référencé 140, aussi également filiforme, définissant aussi une première extrémité dite distale 144 et une deuxième extrémité dite proximale 146 , pouvant être rendu solidaire par ladite extrémité proximale 146 au moins provisoirement avec au moins une autre extrémité 24 dudit premier élément de soutènement 20 allongé, ladite extrémité distale 144 du deuxième élément de traction 140 étant aussi prévue pour constituer au moins un deuxième système de fixation ou d'ancrage 148 sur une autre paroi ou une autre partie de paroi P2 ou des tissus résistants P du corps du mammifère, également activé par une traction exercée sur le deuxième élément de traction 140 , notamment au niveau de son extrémité proximale 146.

Selon un mode de réalisation particulièrement avantageux de l'invention, le matériau sensiblement non extensible, souple, déformable, utilisé pour réaliser soit le premier élément de traction, soit le deuxième élément traction, soit chaque élément traction, soit le ou les élément(s) de soutènement, soit l'ensemble de ces éléments, est un polymère organique compatible avec une implantation à l'intérieur des tissus d'un mammifère, ce matériau organique étant avantageusement choisi parmi le polyéthylène, le polypropylène, ou le nylon. Le matériau actuellement préféré est le polypropylène.

Le choix de ce matériau est particulièrement avantageux car il est disponible commercialement à un prix raisonnable sous la forme de fils qui peuvent être réalisés non-tissés ou tissés ou tricotés pour se présenter sous la forme d'une bandelette, comme cela est bien visible notamment aux figures 1 à 6, dont la longueur et la largeur peuvent être définie(s) de manière indépendante, à volonté, et ainsi être adaptées pratiquement à chaque patient. De telles bandelettes sont généralement disponibles dans le commerce et peuvent être découpées à volonté directement par le chirurgien. Elles offrent en outre l'avantage de pouvoir être traversées ou perforées facilement par un outil de solidarisation 106 tel qu'un élément formant aiguille.

Ainsi, selon une variante de réalisation particulière, on peut prévoir que le premier élément de soutènement 20, ou chaque élément de soutènement, présente la forme d'une bandelette 22 ayant une largeur suffisante pour réaliser un soutènement sûr et fiable de l'organe à soutenir. On entend par o «largeur suffisante» de l'élément de soutènement, une largeur qui assure le soutènement de l'organe à soutenir sans risque de rupture ou de blessure de cet organe comme cela pourrait être le cas si la largeur de l'élément de soutènement est trop faible. Par exemple, l'élément de soutènement 20 sous forme de bandelette peut présenter une longueur d'environ 2,5 cm et une largeur d'environ 1 cm.

Selon une autre variante de réalisation particulière de l'invention, on peut prévoir que le premier élément de traction ou le deuxième élément de traction, ou les deux, comprenne, ou soit constitué de, une bandelette 42, 142 de largeur plus faible que la largeur de la bandelette 22 de l'élément de soutènement 20. En particulier, cette largeur de la bandelette 42, 142 de l'élément de traction 40, 140 peut être inférieure ou égale à environ la moitié de la largeur de la bandelette de l'élément de soutènement. Ainsi, la longueur de chaque élément de traction pourra être d'environ 10 cm, avant ou après la formation des replis 49, des systèmes d'ancrage 48, 148 par exemple formés comme décrit précédemment selon le principe de la couture avec passage d'une aiguille au travers de la bandelette, et présenter une largeur au plus d'environ 0,5 cm, soit 5 mm.

Selon encore une autre variante de réalisation particulière de l'invention, il est prévu au niveau de chaque extrémité 24, 26 de chaque élément de soutènement 20 au moins un élément de solidarisation 30, 32 d'au moins une extrémité proximale 46, 146 d'au moins un élément de traction 40, 140.

Selon une réalisation particulière, avantageuse de l'invention, cet élément de solidarisation 30, 32 comprend, ou est constitué de, une fente 31, 33, réalisée dans le matériau de l'élément de soutènement 20, cette fente 31, 33, voir figure 1, étant selon une réalisation particulière disposée sensiblement parallèlement aux bords longitudinaux de ladite bandelette 22. La dimension de cette fente peut varier dans de larges limites. Cependant, la dimension de cette fente sera limitée au minimum pour permettre le passage de l'extrémité proximale de l'élément de traction en vue de sa solidarisation avec l'élément de soutènement.

La solidarisation de l'élément de traction 40, 140 avec l'élément de soutènement 20 peut être réalisée très simplement, comme montré symboliquement respectivement à la figure 6 et à la figure 11. Lorsque l'élément de traction 40, 140 et l'élément de soutènement 20 sont formés chacun d'une bandelette sous forme non tissée ou tissée à partir de fils de matériau organique polymère précité, le chirurgien procède à l'insertion de l'extrémité proximale 46,146 de l'élément de traction 40, 140 au travers de la fente respective, 31 ou 33, puis à son repli et application de l'extrémité 46, 146 de l'élément de traction contre la surface en regard soit de l'élément de soutènement 20, soit de la surface de l'élément de traction 40, 140, en réalisant ainsi une solidarisation de leurs surfaces respectives par imbrication naturelle dans les mailles de la bandelette non tissée ou tissée, en obtenant de manière particulièrement simple et aisée à réaliser, un effet de fixation de type Velcro, bien connu de l'homme de l'art.

Un deuxième mode de réalisation des éléments principaux du dispositif chirurgical selon la présente invention est représenté de manière éclatée à la figure 12, et est similaire à celui de la figure 1. De ce fait, il est utilisé les mêmes numéros de référence que ceux de la figure 1 pour les éléments identiques ou similaires mais augmentés ici de 200. Ainsi, le dispositif 210 comprend au moins un premier élément de soutènement 220, de forme allongée, par exemple ici également sous forme d'une bandelette, et au moins un premier élément de traction 240 avantageusement filiforme, dont l'extrémité distale sera modifiée comme montré aux figures 13, 13A, pour comprendre ou constituer un premier système de fixation ou d'ancrage 248, de manière similaire au mode de réalisation des figures 1à 6. Dans ce mode de réalisation, les éléments de solidarisation 230, 232 peuvent comprendre des orifices ou fentes ayant une section de forme quelconque mais représenté ici de manière simple sous forme d'orifices ou fentes de section circulaires 231, 233. On comprend que ce deuxième mode de réalisation présente les mêmes avantages que celui représenté aux figures 1 à 11 ;
- les figures 13 et 13A, représentent les étapes successives de fabrication du système de fixation ou d'ancrage 248 à l'extrémité 244 de chaque élément de traction , ici portant le numéro de référence générale 240 de manière similaire aux Figures 2A à 2C, par la formation de plis 249 à l'extrémité de 244, figure 13, puis leur perforation à l'aide du système à aiguille 306, qui sera enlevée ultérieurement, figure 13A ;
- la figure 14 présente un troisième mode de réalisation d'un dispositif chirurgical selon la présente invention, pour lequel les chiffres de référence ont encore été augmentés de 100 par rapport au deuxième mode de réalisation de la figure 12. Selon ce troisième mode de réalisation, l'élément de soutènement 320 de forme allongée, par exemple ici sous la forme d'une bandelette, est réalisé monobloc avec un premier élément de traction 340, ce premier élément de traction 340 étant réalisé de manière similaire à celui des deux premiers modes de réalisation représentés aux figures 1 et 12. Dans ce cadre, on comprend que par le mode de relation préféré où l'élément de soutènement 320 et l'élément de traction 340 sont sous forme de bandelette tissée ou tricotée, l'élément de soutènement 320 est réalisé en une seule étape lors de la fabrication de l'élément de traction 340 en continuité avec celui-ci. Dans ce mode de réalisation, l'élément support 320 peut être simplifié pour comprendre sur une seule extrémité libre, au moins un organe de solidarisation, par exemple un orifice ou fente 333 ;
- les figures 15 et 15A, représentent les étapes successives de fabrication du système de fixation ou d'ancrage 348 à l'extrémité 344 de chaque élément de traction 340 , par la formation de plis 349, de manière similaire aux Figures 2A à 2C ;
- la figure 16 représente, de manière éclatée, un quatrième mode de réalisation d'un dispositif chirurgical selon la présente invention, dans lequel l'élément de soutènement 420 est particulièrement adapté pour réaliser le soutènement du bulbe urétral d'un mammifère mâle, de préférence un homme, et dans ce cas, il est préféré que l'élément de soutènement soit de plus grande largeur pour assurer un soutien ferme et fiable du bulbe urétral mâle. Selon ce mode de réalisation, l'élément de soutènement 420 comprend de préférence de chaque côté 424, 426 au moins un et encore de préférence au moins deux organes de solidarisation, tels que orifices ou fentes 431, 433, de manière à simplifier la procédure de solidarisation de l'élément de soutènement avec les éléments de traction 440, identiques ou similaires à ceux objets des modes de réalisation précédents représentés aux figures 1 à 15 ;
- la figure 17 représente le dispositif chirurgical de la figure 16 sous forme assemblée, permettant de voir la facilité d'assemblage. On observera que grâce à la combinaison des deux moyens de solidarisation 431 ou 433 de chaque côté de l'élément de soutènement 420, formé par exemple ici sous forme de bande tissée ou tricotée, après insertion de l'extrémité libre 446, opposé au système de fixation 448, obtenu par la formation de plis et leur perforation de manière similaire à la méthode de fabrication montrée aux figures 2A, 2B, 2C, il est obtenu une solidarisation sur et fiable des éléments de traction 440 avec l'élément de soutènement 420, sans avoir à utiliser un système de fixation complexe et difficile à manipuler ;
- la figure 18 représente la première étape de la procédure chirurgicale d'implantation chez un mammifère mâle , de préférence un homme, du dispositif chirurgical représenté à la figure 16, montrant la ligne d'incision transversale I entre le rectum R et les parties génitales G, le plus loin possible du rectum R. Il est représenté à la figure 18 le bassin B en traits fantômes, ainsi que les trous obturateurs H;
- la figure 19 représente la situation obtenue après la première étape de la procédure chirurgicale, après dégagement des chairs , permettant de bien observer la présence des trous obturateurs H du bassin, avec de manière schématique l'accès à chaque membrane musculaire M obturant chaque trou obturateur H du bassin B. Le bulbe urétral U à soutenir est clairement visible et son accès apparent.
- la figure 20 représente l'étape suivante de la procédure chirurgicale, similaire dans son principe à celle de la chirurgie d'implantation chez un mammifère femelle, selon laquelle chaque système de fixation 440 de la figure 16 a été mis en place par perforation de la membrane musculaire M au niveau du trou obturateur H, à l'aide d'un trocart perforateur, similaire à celui des figures 4, 5, 7, 8, et traction en retrait pour déployer le système de fixation 448 pour qu'il prenne une forme de parapluie, reposant sur une grande surface contre la membrane musculaire M, de manière similaire à la mise en place chez un mammifère femelle comme représenté aux figures 7 à 9, comme cela est bien compréhensible pour l'homme de l'art.
- la figure 21 représente la fin de la mise en place chez un mammifère mâle, ici un homme, du dispositif chirurgical selon l'invention, comprenant l'étape de solidarisation de l'élément de soutènement 420 avec les éléments de traction 440 de manière similaire à la position de solidarisation représentée à la figure 17, et traction sur au moins un, de préférence les deux, éléments de traction 440, selon le niveau de traction, donc de soutien du bulbe urétral, souhaité par le chirurgien, afin de traiter de manière sûre, fiable et durable le problème de l'incontinence masculine du patient. La position du dispositif est bien visible sur les vues de face et de côté des figures 22 et 23.

Selon un deuxième aspect, la présente invention concerne aussi en tant que produit nouveau indépendamment brevetable chaque élément de traction 40, 240, 340, 440 comprenant une extrémité distale 44, 244, 344 ou 444 comprenant ou constituant un système de fixation ou d'ancrage 48, 248, 348 ou 448 tel que précédemment défini ou tel que résultant de la description suivante faite en référence aux dessins qui font partie intégrante de la présente invention et qui complètent donc la présente description.

Selon un troisième aspect, la présente invention couvre encore un kit de soutènement et de fixation ou d'ancrage, caractérisé en ce qu'il comprend au moins un dispositif chirurgical 10, 210, 310 ou 410 formant prothèse chirurgicale selon l'un quelconque des aspects précédents, ainsi qu'un dispositif introducteur (100) avantageusement sous forme d'un trocart (102) de pénétration, avec présence d'un élément poussoir (104) de l'élément de traction 40, 240, 340 ou 440, avec son extrémité distale comprenant ou constituant le système de fixation ou d'ancrage 48 ou 248, 348 ou 448, monté de manière compacte ou repliée à l'intérieur du trocart de pénétration (102). Avantageusement, le trocart (102) ou un élément poussoir (180) peut comporter un élément de blocage anti- retrait de l'élément de traction en place à l'intérieur du trocart. On peut prévoir avantageusement un élément formant butée d'arrêt 105 de poussée en avant de l'élément poussoir 104, afin de ne pas pénétrer trop loin, après sortie du dispositif de fixation ou d'ancrage 48 du trocart 102, comme montré à la figure 5.

Selon un quatrième aspect, la présente invention couvre encore une méthode chirurgicale pour réaliser le soutènement d'un organe à soutenir d'un mammifère en ayant besoin, caractérisée en ce qu'elle comprend :
a) une anesthésie locale au voisinage de l'organe à soutenir U sur le futur trajet de la prothèse, opération classique évidemment non représentée ;
b) une incision I et une dissection des tissus en regard et de part et d'autre de l'organe U à soutenir dudit mammifère, opération classique non représentée ;
c) la mise en place d'un premier dispositif introducteur 100 formant trocart 102 dans le passage créé par la dite incision et dissection des tissus, sur un côté et au-delà de l'organe à soutenir, et après franchissement par perforation à l'aide du dispositif introducteur formant trocart 102 du support relativement au déchirement dudit mammifère, tel que os ou tissus appropriés comme par exemple la paroi abdominale ou pelvienne transversale P, par exemple à la position P1, ou encore la membrane musculaire M du trou obturé H du bassin, le dispositif introducteur formant trocart et ayant une forme tubulaire creuse comprenant en son intérieur un premier élément de traction 40 précité, ainsi qu'un élément poussoir 104 ;
d) une action de poussée sur l'élément poussoir 104 de manière à faire sortir du dispositif introducteur l'extrémité du premier élément de traction 40, 240, 340 ou 440 jusqu'à ce que au moins le système de fixation 48, 248, 348 ou 448 de l'élément de traction soit libéré au-delà dudit support P relativement résistant au déchirement ;
e) après la libération ou largage du système de fixation 48, 248, 348 448, une traction au voisinage de la partie proximale de l'élément de traction, permettant le positionnement et l'ouverture ou déploiement par effet d'écrasement de l'extrémité distale de l'élément de traction formant système de fixation ou d'ancrage, avec obtention d'un positionnement dit en parapluie présentant une grande surface de fixation d'ancrage contre le dit support P relativement résistant au déchirement dudit mammifère, comme montré à la figure 8 ;
f) le retrait du trocart de pénétration 102, laissant en place le système d'ancrage s'appuyant sur le support P ou M choisi pouvant ainsi jouer son rôle de point de fixation car la taille du système de fixation ou d'ancrage excède largement celle de l'orifice provoqué par la pénétration du trocart, comme montré à la figure 9 ou à la figure 20 ou 21 ;
g) la répétition des étapes précédentes c), d), e), f) de l'autre côté de l'organe pour introduire un deuxième élément de traction 140 ou 240, 340 ou 440 , de préférence identique au premier élément traction 40 , par exemple à la zone P2 ou M ;
h) lorsque aucun élément de traction n'est initialement prévu solidarisé de l'élément de soutènement, la solidarisation à l'aide de l'outil 106 de l'extrémité proximale du premier ou deuxième élément de traction avec un élément de solidarisation 30 en regard d'une première extrémité d'un élément de soutènement allongé 20, de manière à réaliser un premier mouvement de traction sur l'extrémité proximale élément de traction pour un pré-positionnement, comme montré à la figure 10 ;
i) la réalisation de la même procédure que les étapes précédentes concernant l'autre deuxième 140 ou premier élément de traction avec un autre élément de solidarisation 32 en regard d'une deuxième extrémité du même élément de soutènement allongé, jusqu'à mise en tension dudit élément de soutènement allongé en position appropriée avec ledit organe pour réaliser le soutien recherché dudit organe, comme montré la figure 11, l'enlèvement des outils 106 et des éléments de blocage 108 ;
j) la fermeture ou suture de l'incision avec un moyen de fermeture chirurgicale appropriée, tel que fil ou agrafe résorbable, opérations classiques non représentées.

En référence maintenant à la figure 14, l'élément de soutènement peut être fabriqué solidaire ou monobloc ou intégral à un premier élément de traction, tel que l'élément de traction 40, 140 ou 240. Dans ce cas, la méthode chirurgicale est simplifiée dans la mesure où l'étape h n'a lieu qu'avec le deuxième élément de traction puisque le premier élément de traction est déjà solidarisé à l'élément de soutènement. Ainsi, les étapes h et i se réalisent en une seule étape.

Selon un premier mode de réalisation préféré de ce procédé chirurgical, celui-ci est utilisé pour réaliser le traitement de l'incontinence d'un mammifère femelle, en particulier d'une femme. Dans ce cadre, l'organe à soutenir est l'urètre U, de préférence au voisinage de la partie urétrale proche de la vessie, comme représenté aux figures 7 à 11. Dans ce cadre, il est préféré selon la présente invention que l'extrémité distale de l'élément de traction prenne appui sur les tissus de la paroi pelvienne P, de préférence des tissus sensiblement transversaux de la paroi pelvienne dans la partie située entre l'urètre et les côtés du mammifère femelle, en particulier une femme, au-dessus de la paroi vaginale, comme représenté aux figures 7 à 11.

L'homme de l'art comprend que avec la procédure chirurgicale de l'invention, à la fin de l'étape g), les deux ancrages latéraux sont en place et les deux extrémités libres 46,146 des éléments de traction 40,140 ressortent par l'incision chirurgicale.

Il est aisé pour le chirurgien de passer l'extrémité libre 46 ou 146 des éléments de traction et de positionner l'élément de soutènement 20 en la faisant coulisser au travers des fentes 31,33. L'invention permet ainsi un positionnement idéal de l'élément de soutènement 20 ou prothèse en ajustant très simplement la tension et en permettant de réduire un excès de tension en retirant légèrement l'élément de soutènement 20. Il est également possible d'envisager une reprise chirurgicale dans les 10 jours suivants pour détendre une traction trop importante, ce qui est très difficile ou beaucoup plus aléatoire avec les procédés chirurgicaux antérieurement utilisés.

Après le choix définitif de la position idéale, l'opérateur sectionne l'excès de longueur de chaque élément de traction 40 ou 140, de préférence en laissant environ 2 cm en dessous de l'élément de soutènement 20 pour replier l'extrémité libre 46 ou 146 de 180° le long de l'élément de traction 40 ou 140 comme représenté à la figure 6 et à la figure 11, sans réaliser de suture parce que l'invention permet une fixation suffisante grâce à la structure en bandelette tissée ou non tissée de l'élément d'ancrage ou de l'élément de soutènement 20 , par effet de type Velcro, rendu possible par les qualités d'adhérence du maillage de la prothèse. La cicatrisation naturelle va définitivement bloquer la prothèse par fibrose en environ deux semaines.

Selon le mode de réalisation représenté aux figures 16 à 23, l'invention couvre encore une méthode chirurgicale pour réaliser le soutènement d'un organe à soutenir d'un mammifère mâle, en particulier d'un homme. Dans ce cas, on traitera généralement l'incontinence mâle, en particulier après opération de la prostate. Dans ce cadre, l'organe à soutenir est le bulbe urétral U, voir figures 19 à 23, de préférence entre les orifices obturés H du bassin B,c'est-à-dire entre les os ischion du bassin B, comme représenté aux figures 18 à 23. Dans ce cadre, il est encore préféré selon la présente invention que l'extrémité distale 448 de chaque élément de traction 440 prenne appui sur les tissus de la membrane musculaire M obturant les trous obturés H du bassin B , comme cela est bien visible aux figures 20 et 21.

L'homme de l'art comprend qu'avec la procédure chirurgicale de l'invention, telle que définie pour un mammifère femelle, de préférence une femme, pour un mammifère mâle la procédure de solidarisation avec l'élément de soutènement peut être simplifiée comme montré aux figures 20 et 21, en raison d'une plus grande accessibilité du bulbe urétral U chez l'homme, par rapport à l'intérieur du vagin chez la femme, en réalisant une incision sensiblement transversale ou horizontale entre le rectum R et les parties génitales G, le plus loin du rectum R. En outre, la procédure de solidarisation peut être réalisée facilement par la présence de deux éléments de solidarisation constitués ici par deux orifices ou fentes 431, 433 décalés à chaque extrémité de l'élément de soutènement tel qu'une bandelette, et qui réalise un effet de solidarisation très efficace entre l'élément de soutènement et les éléments de traction, sans avoir nécessairement à réaliser le repliage comme prévu dans le cadre des modes de réalisation précédents pour la femme, et sans prévoir de suture. Evidemment, cette solution peut aussi être réalisée dans le cadre de soutènement de l'urètre féminin.

L'invention ne se limite pas au soutènement de l'urètre féminin ou du bulbe urétral masculin, mais peut être utilisée pour d'autres organes ptosés, par exemple le prolapsus génital de la femme, éventuellement le soutènement de la vessie ou encore du rectum.

Dans le cadre de l'invention, un sondage post-opératoire n'est pas recommandé car inutile et source de risque infectieux. Les procédés antérieurs à l'invention imposent le plus souvent un sondage de la vessie à cause des douleurs et des anesthésies plus profondes, risques qui sont évités avec l'invention. L'invention apporte donc une grande simplicité de conception de prothèse, une simplification de la procédure chirurgicale ce qui constitue des avantages particulièrement inattendus et non évidents pour l'homme de l'art.

L'invention comprend évidemment tous les moyens constituant des moyens techniques équivalents aux moyens décrits et représentés aux figures annexées qui font partie intégrante de la présente invention et complètent ainsi la présente description.

Chaque caractéristique qui apparaît être nouvelle par rapport à un ETAT DE LA TECHNIQUE quelconque est revendiquée en tant que telle dans sa fonction et en tant que moyen général.

## Revendications

1. Dispositif chirurgical (10) formant prothèse chirurgicale, destiné au soutènement d'un organe biologique à soutenir (U) d'un mammifère, notamment à traiter l'incontinence urinaire, **caractérisé en ce qu'**il comprend :
a) au moins un premier élément de soutènement (20) comprenant un élément de forme allongée (22) définissant une première (24) et une deuxième (26) extrémité pour exercer une action de soutènement dudit organe biologique (U) ; ledit premier élément (22) de forme allongée étant réalisé au moins en partie en un matériau sensiblement non extensible, souple, déformable ;
b) au moins un premier élément de traction (40), avantageusement filiforme, définissant une première extrémité (44) dite distale et une deuxième extrémité (46), dite proximale, pouvant être rendu solidaire par ladite extrémité proximale (46) au moins provisoirement avec au moins une extrémité (26) dudit premier élément (20) de soutènement allongé, ladite extrémité distale (44) étant prévue pour constituer au moins un premier système de fixation ou d'ancrage (48) activé par une traction exercée sur le premier élément de traction notamment au niveau de son extrémité proximale ; **caractérisée en ce que** ledit système de fixation (48) prévu à l'extrémité distale de l'élément de traction comprend au moins un repli (49), de préférence plusieurs replis, de l'élément de traction (40) qui, lors d'une action de traction exercée sur l'élément de traction, est déformé ou écrasé pour se déployer en imitant un effet de parapluie définissant une surface suffisante de fixation ou d'ancrage.

2. Dispositif selon la revendication 1, **caractérisé en ce que** les replis (49) sont solidarisés entre eux par perforation par un élément formant aiguille, de préférence sensiblement en leur milieu, pour constituer ainsi le système de fixation ou d'ancrage 48.

3. Dispositif selon la revendication 1 ou 2, **caractérisé en ce que** le premier élément de traction (40) est aussi réalisé au moins en partie en un matériau sensiblement non extensible, souple, déformable, en particulier en le même matériau que celui utilisé pour réaliser le premier élément de soutènement (20).

4. Dispositif selon l'une des revendications 1 à 3, **caractérisé en ce qu'**il comprend un deuxième élément de traction (140), de préférence de même conception que le premier élément de traction (40), ainsi également filiforme, définissant aussi une première extrémité (144) dite distale et une deuxième extrémité (146) dite proximale, pouvant être rendu solidaire par ladite extrémité proximale (144) au moins provisoirement avec au moins une autre extrémité (24) dudit premier élément de soutènement (20) allongé, ladite extrémité distale (144) du deuxième élément de traction (140) étant aussi prévue pour constituer au moins un deuxième système de fixation ou d'ancrage (148) sur une autre paroi ou une autre partie (P2) de paroi (P) ou des tissus résistants du corps du mammifère, également activé par une traction exercée sur le deuxième élément de traction (140), notamment au niveau de son extrémité proximale (146).

5. Dispositif selon l'une des revendications 1 à 4, **caractérisé en ce que** le matériau sensiblement non extensible, souple, déformable, utilisé pour réaliser soit le premier élément de traction (40), soit le deuxième élément traction (140) ou chaque élément de traction (40, 140), soit le ou chaque élément(s) de soutènement (20), soit l'ensemble de ces éléments, est un polymère organique compatible avec une implantation à l'intérieur des tissus d'un mammifère, ce matériau organique étant avantageusement choisi parmi le polyéthylène, le polypropylène, ou le nylon, le matériau actuellement préféré étant le polypropylène.

6. Dispositif selon l'une des revendications 1 à 5, **caractérisé en ce que** le premier élément de soutènement (20), ou chaque élément de soutènement (20), présente la forme d'une bandelette (22) ayant une largeur suffisante pour réaliser un soutènement sûr et fiable de l'organe (U) à soutenir.

7. Dispositif selon l'une des revendications 1 à 6, **caractérisé en ce que** le premier élément de traction (40) ou le deuxième élément de traction (140), ou les deux, comprend, ou est constitué de, une bandelette (42, 142) de largeur plus faible que la largeur de la bandelette de l'élément de soutènement ; en particulier, cette largeur de la bandelette de l'élément de traction pouvant être inférieure ou égale à environ la moitié de la largeur de la bandelette (22) de l'élément de soutènement (20).

8. Dispositif selon l'une des revendications 1 à 7, **caractérisé en ce qu'**il est prévu au niveau de chaque extrémité (24, 26) de chaque élément de soutènement (20) au moins un élément de solidarisation (30, 32) d'au moins une extrémité proximale d'au moins un élément de traction (40, 140).

9. Dispositif selon la revendication 8, **caractérisé en ce que** cet élément de solidarisation (30, 32) comprend, ou est constitué de, une fente (31, 33) réalisée dans le matériau de l'élément de soutènement (20), cette fente (31, 33) étant selon une réalisation particulière disposée sensiblement parallèlement aux bords longitudinaux de ladite bandelette (22).

10. Dispositif selon l'une des revendications précédentes, **caractérisé en ce que** l'élément de traction (40, 140) et l'élément de soutènement (20) sont formés chacun d'une bandelette non tissée, ou tissée, réalisée à partir de fils de matériaux organiques polymères précités ; l'élément de traction (40, 140) et de l'élément de soutènement (20) sont solidarisés à l'aide d'au moins un élément de solidarisation (30) et par application de l'élément de traction (40, 140) contre la surface en regard de l'élément de soutènement, en réalisant une solidarisation de leurs surfaces respectives par imbrication naturelle obtenant un effet de fixation de type velcro.

11. Dispositif selon l'une des revendications précédentes, **caractérisé en ce que** l'élément de soutènement (310) est réalisé monobloc avec l'un des dés élément de traction précités (340), et peut ainsi ne comprendre qu'au moins un moyen de solidarisation tel qu'un orifice ou fente (333), en permettant ainsi lorsque l'élément de soutènement et l'élément de traction sont réalisés sous forme de bandelette, une fabrication de ceux-ci en une seule étape.

12. Dispositif selon l'une des revendications précédentes, **caractérisé en ce qu'**il est prévu pour un traitement de l'incontinence urinaire, en particulier d'un mammifère femelle, en particulier une femme, ou d'un mammifère mâle, en particulier un homme.

13. Elément de traction (40, 240, 340, 440) destiné à être mis en oeuvre avec un dispositif chirurgical selon l'une quelconque des revendications 1 à 12, ledit élément de traction (40, 240, 340, 440) étant avantageusement filiforme, définissant une extrémité de suspension comprenant ou constituant un système de fixation ou d'ancrage (48, 248, 348, 448), et étant tel que défini à l'une quelconque des revendications de dispositif 1 à 12.

14. Kit de soutènement et de fixation ou d'ancrage, **caractérisé en ce qu'**il comprend au moins un dispositif chirurgical (10, 210, 310,410) formant prothèse chirurgicale, tel que défini à une quelconque des revendications 1 à 12 ; ainsi qu'un dispositif introducteur (100) avantageusement sous forme d'un trocart (102) de pénétration, avec présence d'un élément poussoir (104) de l'élément de traction (40, 240, 340, 440) avec son extrémité distale comprenant ou constituant le système de fixation ou d'ancrage (48, 248, 348, 448), monté de manière compacte ou repliée à l'intérieur du trocart (102) de pénétration.

## Claims

1. A surgical device (10), forming a surgical prosthesis, for supporting a biological organ (U) of a mammal in need of support, notably to treat urinary incontinence, **characterized in that** it comprises:
a) at least one first support element (20) comprising an element of elongate shape (22) defining a first end (24) and a second end (26) for exerting a supporting action on said biological organ (U); said first element of elongate shape (22) being made at least in part out of a material that is substantially non-extensible, flexible, and deformable; and
b) at least one first traction element (40), advantageously a filiform element, defining a first end (44) referred to as a distal end and a second end (46) referred to as a proximal end, which elements can be fastened by said proximal end (46) at least temporarily to at least one end (26) of said first elongate support element (20), said distal end (44) being provided to constitute at least one first fixing or anchor system (48), activated by traction exerted on the first traction element, in particular at its proximal end; **characterized in that** said fixing system (48) provided at the distal end of the traction element comprises at least one fold (49), preferably several folds, in the traction element (40)which, when traction is exerted on the traction element, becomes reformed or flattened so as to deploy while imitating an umbrella effect so as to define an area that is sufficient for fixing or anchoring.

2. A device according to claim 1, wherein the folds (49) are fastened together, by being perforated by a needle-forming element, thereby constituting the fixing or anchor system (48).

3. A device according to claim 1 or 2, wherein the first traction element (40) is also made at least in part out of a material that is substantially non-extensible, flexible, and deformable, in particular out of the same material as that used for making the first support element (20).

4. A device according to one of claims 1 to 3, comprising a second traction element (140), preferably of the same design as the first traction element (40), and is thus likewise filiform, likewise defining a distal first end (144) and a proximal second end (146), being suitable for being fastened by said proximal end (144) at least temporarily to at least one other end (24) of said first elongate support element (20), said distal end (144) of the second traction element (140) being also provided to constitute at least one second fixing or anchor system (148) against another wall or another portion (P2)of a wall (P) or of tear-resistant tissue of the mammalian body, likewise activated by exerting traction on the second traction element (140), in particular via its proximal end (146).

5. A device according to one of claims 1 to 4, wherein the substantially non-extensible, flexible, and deformable material used for making either the first traction element (40), or the second traction element (140), or each traction element (40, 140), or the or each support element 20), or all of these elements, is an organic polymer compatible with being implanted in mammalian tissue, the organic material being advantageously selected from polyethylene, polypropylene, and nylon, the presently-preferred material being polypropylene.

6. A device according to one of claims 1 to 5, wherein the first support element (20), or each support element (20), presents the form of a strip (22) of width that is sufficient to achieve certain and reliable support of the organ (U) to be supported.

7. A device according to one of claims 1 to 6, wherein the first traction element (40) or the second traction element (140), or both of them, comprises or is constituted by a strip (42, 142) of width that is narrower than the width of the strip of the support element; in particular said width of the strip of the traction element can be less than or equal to about half the width of the strip (22) of the support element (20).

8. A device according to one of claims 1 to 7, wherein there is provided at each end (24, 26) of each support element (20), at least one fastener element (30, 32) of at least one proximal end of at least one traction element (40, 140).

9. A device according to claim 8, wherein the fastener element (30, 32) comprises or is constituted by a slot (31, 33) made in the material of the support element (20), said slot (31, 33) in a particular embodiment being disposed substantially parallel to the longitudinal edges of said strip (22).

10. A device according to one of preceding claims, wherein traction element (40, 140) and the support element (20) are each formed by a woven or non-woven strip made from the said yarns of organic polymer material; the traction element (40, 140) and the support element (20) are fastened together using at least one fastener element (30) by pressing the traction element (40, 140) against the facing surface of the support element, causing their respective surfaces to be fastened together by natural interpenetration obtaining a Velcro type fixing effect.

11. A device according to one of the preceding claims, wherein said support element (310) is monobloc with one of said traction elements (340), and can thus comprise only at least one hastening means such as an orifice or a slot (333) thus enabling when the support element and the traction element are implemented in the form of a strip, a manufacture thereof in a single step.

12. A device according to any one of the preceding claims, **characterized in that** it is foreseen for the treatment of urinary incontinence, in particular of female mammal, in particular a woman, or for a male mammal, in particular a man.

13. A traction element (40, 240, 340, 440) for implementing with a surgical device according to any one of claims 1 to 12, said traction element (40, 240, 340, 440) being advantageously filiform, defining a suspension point comprising or constituting a fixing or anchor system (48, 248, 348, 448), and being as defined in any one of device claims 1 to 12.

14. A support and fixing or anchor kit comprising at least one surgical device (10, 210, 310, 410) forming a surgical prosthesis as defined in any one of claims 1 to 12; together with an introducer device (100) advantageously in the form of a penetration trocar (102), with the presence of a pusher element (104) for pushing the traction element (40, 240, 340, 440) with its distal end comprising or constituting the fixing or anchor system (48, 248, 348, 448), mounted in compact manner or folded inside the penetration trocar (102) .

## Patentansprüche

1. Chirurgische Vorrichtung (10), die eine chirurgische Prothese bildet, die dazu bestimmt ist, ein biologisches Organ (U) eines Säugetiers zu stützen, insbesondere zur Behandlung der Harninkontinenz,
**dadurch gekennzeichnet, daß** sie aufweist:
a) mindestens ein erstes Stützelement (20), das ein Element in länglicher Form (22) aufweist, das ein erstes (24) und ein zweites (26) Ende definiert, um eine Stützwirkung für das biologische Organ (U) auszuüben, wobei das erste längliche Element (22) mindestens zum Teil aus einem im wesentlichen nicht dehnbaren, biegsamen, verformbaren Material hergestellt ist,
b) mindestens ein erstes Zugelement (40), das vorteilhafterweise fadenförmig ist, das ein erstes Ende (44), das distal genannt wird, und ein zweites Ende (46), das proximal genannt wird, definiert, das über das proximale Ende (46) mindestens vorübergehend mit mindestens einem Ende (26) des ersten länglichen Stützelements (20) verbunden werden kann, wobei das distale Ende (44) dazu vorgesehen ist, mindestens ein erstes Befestigungs- oder Verankerungssystem (48) zu bilden, das durch einen Zug aktiviert wird, der auf dem ersten Zugelement insbesondere auf dem Niveau seines proximalen Endes ausgeübt wird, **dadurch gekennzeichnet, daß** das Befestigungssystem (48), das an dem distalen Ende des Zugelements vorgesehen ist, mindestens einen Umschlag (49), vorzugsweise mehrere Umschläge, aufweist, die bei einer Zugwirkung, die an dem Zugelement ausgeübt wird, verformt oder zerdrückt werden, um sich wie ein Regenschirm, der eine ausreichende Befestigungs- oder Verankerungsfläche definiert, aufzuspannen.

2. Vorrichtung nach Anspruch 1, **dadurch gekennzeichnet**, die Umschläge (49) untereinander durch mit einem Element, das eine Nadel bildet, vorzugsweise im wesentlichen in ihrer Mitte verbunden sind, um ein Befestigungs- oder Verankerungssystem (48) zu bilden.

3. Vorrichtung nach Anspruch 1 oder 2, **dadurch gekennzeichnet, daß** das erste Zugelement (40) auch mindestens zum Teil aus einem im wesentlichen nicht dehnbaren, biegsamen, verformbaren Material hergestellt ist, insbesondere aus dem Material, das zum Herstellen des ersten Stützelements (20) verwendet wird.

4. Vorrichtung nach einem der Ansprüche 1 bis 3, **dadurch gekennzeichnet, daß** sie ein zweites Zugelement (140) aufweist, vorzugsweise mit der gleichen Konzeption wie das erste Zugelement (40), daher ebenfalls fadenförmig, aufweist, das ebenfalls ein erstes Ende (144), das distal genannt wird, definiert, und ein zweites Ende (146), das proximal genannt wird, das durch das proximale Ende (144) mindestens vorübergehend mit mindestens einem anderen Ende (24) des ersten länglichen Stützelements (20) verbunden werden kann, wobei das distale Ende (144) des zweiten Zugelements (140) auch vorgesehen ist, um mindestens ein zweites Befestigung- oder Verankerungssystem (148) auf einer anderen Wand oder einem anderen Teil (P2) der Wand (P) oder der widerstandsfähigen Gewebe des Körpers des Säugetiers zu bilden, das ebenfalls durch einen Zug aktiviert wird, der an dem zweiten Zugelement (140), insbesondere auf dem Niveau seines proximalen Endes (146) ausgeübt wird.

5. Vorrichtung nach einem der Ansprüche 1 bis 4, **dadurch gekennzeichnet, daß** das im wesentlichen nicht dehnbare, biegsame, verformbare Material, das zum Herstellen entweder des ersten Zugelements (40) oder des zweiten Zugelements (140) oder jedes Zugelements (40, 140), oder des oder jedes Stützelements (20) oder aller dieser Elemente verwendet wird, ein organisches Polymer ist, das mit einem Implantieren im Inneren der Gewebe eines Säugetiers kompatibel ist, wobei dieses organische Material vorteilhafterweise aus Polyethylen, Polypropylen und Nylon ausgewählt ist, wobei das derzeit bevorzugte Material Polypropylen ist.

6. Vorrichtung nach einem der Ansprüche 1 bis 5, **dadurch gekennzeichnet, daß** das erste Stützelement (20) oder jedes Stützelement (20) die Form eines Bands (42) aufweist, das eine ausreichende Breite hat, um ein sicheres und zuverlässiges Stützen des zu stützenden Organs (U) zu verwirklichen,

7. Vorrichtung nach der Ansprüche 1 bis 6, **dadurch gekennzeichnet, daß** das erste Zugelement (40) oder das zweite Zugelement (140) oder beide ein Band (42, 142) aufweisen oder aus diesem bestehen, das eine kleinere Breite hat als die Breite des Bands des Stützelements, wobei diese Breite des Bands des Zugelements insbesondere kleiner oder gleich etwa der Hälfte der Breite des Bands (42) des Stützelements (20) sein kann.

8. Vorrichtung nach einem der Ansprüche 1 bis 7, **dadurch gekennzeichnet, daß** auf dem Niveau jedes Endes (24, 26) jedes Stützelements (20) mindestens ein Element zum Verbinden (30, 32) mindestens eines proximalen Endes mindestens eines Zugelements (40, 140) vorgesehen ist.

9. Vorrichtung nach Anspruch 8, **dadurch gekennzeichnet, daß** das Element zum Verbinden (30, 33) einen Schlitz (31, 33) aufweist oder aus diesem besteht, der in dem Material des Stützelements (20) hergestellt ist, wobei dieser Schlitz (31, 33) bei einer besonderen Ausführungsform im wesentlichen parallel zu den Längsrändern des Bands (23) anbeordnet ist.

10. Vorrichtung nach einem der vorhergehenden Ansprüche, **dadurch gekennzeichnet, daß** das Zugelement (40, 140) und das Stützelement (20) jeweils aus einem Band aus Vlies oder Gewebe gebildet sind, das ausgehend von den oben genannten Fäden aus organischen Polymermaterialien hergestellt ist, daß das Zugelement (40, 140) und das Stützelement (20) mit Hilfe mindestens eines Elements zum Verbinden (30) und durch Anlegen des Zugelements (40, 140) gegen die Fläche gegenüber dem Stützelement verbunden sind, wobei eine Verbindung ihrer jeweiligen Flächen durch natürliche Verschachtelung, die eine Befestigungswirkung des Typs Velcro ergibt, hergestellt wird.

11. Vorrichtung nach einem der vorhergehenden Ansprüche, **dadurch gekennzeichnet, daß** das Stützelement (310) aus einem Stück mit einem der Zugelemente (340) hergestellt ist und daher eventuell nur ein Mittel zum Verbinden aufweist, wie zum Beispiel eine Öffnung oder einen Schlitz (333), was, wenn das Stützelement und das Zugelement in Form eines Bands hergestellt werden, eine Herstellung beider in einem einzigen Schritt erlaubt.

12. Vorrichtung nach einem der vorhergehenden Ansprüche, **dadurch gekennzeichnet, daß** sie zum Behandeln der Harninkontinenz insbesondere eines weiblichen Säugetiers, insbesondere einer Frau, oder eines männlichen Säugetiers, insbesondere eines Mannes vorgesehen ist.

13. Zugelement (40, 240, 340, 440), das dazu bestimmt ist, mit einer chirurgischen Vorrichtung nach einem der Ansprüche 1 bis 12 umgesetzt werden, wobei das Zugelement (40, 240, 340, 440) vorteilhafterweise fadenförmig ist, indem es ein Anhängeende definiert oder ein Befestigungs- oder Verankerungssystem (48, 248, 348, 448) bildet, indem es wie in einem der Vorrichtungsansprüche 1 bis 12 definiert ist.

14. Bausatz zum Stützen und Befestigen oder Verankern, **dadurch gekennzeichnet, daß** er mindestens eine chirurgische Vorrichtung (10, 210, 310, 440) aufweist, die eine chirurgische Prothese bildet, wie in einem der Ansprüche 1 bis 12 definiert, sowie eine Vorrichtung zum Einführen (100), vorteilhafterweise in Form einer eindringenden Punktionsnadel (102), mit Gegenwart eines Schiebeelements (104) des Zugelements (40, 240, 340, 440) mit seinem distalen Ende, das das Befestigungs- oder Verankerungssystem (48, 248, 348, 448) aufweist oder bildet, kompakt oder zusammengefaltet im Inneren der eindringenden Punktionsnadel (102) installiert.
